# FASCICULE DE BREVET EUROPEEN

(11) **EP 2 536 415 B1**
(45) Date de publication et mention de la délivrance du brevet: **16.07.2014**
(21) Numéro de dépôt: 11705917.0
(22) Date de dépôt: 21.01.2011
(51) Int. Cl.: A61P 1/16, A61P 13/12, A61K 45/06, A61K 33/00

(54) **MÉDICAMENT GAZEUX INHALABLE À BASE D'ARGON CONTRE LES DÉFICIENCES OU DÉFAILLANCES D'ORGANES PÉRIPHÉRIQUES**
INHALIERBARE ARZNEIMITTEL ENTHALTEND ARGONGAS GEGEN STÖRUNGEN VON PERIPHEREN ORGANEN
GASEOUS INHALABLE MEDICAMENT COMPRISING ARGON GAS AGAINST PERIPHERAL ORGAN DEFICIENCIES OR FAILURES

(30) Priorité: 15.02.2010 FR 1051040
(43) Date de publication de la demande: 26.12.2012
(73) Titulaire: L'Air Liquide Société Anonyme pour l'Etude et l'Exploitation des Procédés Georges Claude, 75007 Paris (FR)
(72) Inventeur: LEMAIRE, Marc, F-75014 Paris (FR)
(74) Mandataire: Pittis, Olivier
(86) Numéro de dépôt international: PCT/FR2011/050111
(87) Numéro de publication internationale: WO 2011/098698

(56) Documents cités:
- WO-A1-02/09731
- DE-A1- 10 319 837
- JP-A- 57 212 121
- RU-C1- 2 268 590
- SU-A1- 1 289 437
- US-A- 5 846 556
- PAGEL ET AL: "Cardioprotection by Noble Gases", JOURNAL OF CARDIOTHORACIC AND VASCULAR ANESTHESIA, SAUNDERS, PHILADELPHIA, PA, US, vol. 24, no. 1, 1 février 2010 (2010-02-01), pages 143-163, XP026869285, ISSN: 1053-0770 [extrait le 2009-05-20]
- WAN CHIDAN ET AL: "Experimental study on the cryopreservation of LLC-PK1 epithelial cells with hypoxic UW solution.", JOURNAL OF HUAZHONG UNIVERSITY OF SCIENCE AND TECHNOLOGY. MEDICAL SCIENCES = HUA ZHONG KE JI DA XUE XUE BAO. YI XUE YING DE WEN BAN = HUAZHONG KEJI DAXUE XUEBAO. YIXUE YINGDEWEN BAN AUG 2007 LNKD- PUBMED:17828502, vol. 27, no. 4, août 2007 (2007-08), pages 426-428, XP002584625, ISSN: 1672-0733

## Description

L'invention porte sur l'utilisation d'argon gazeux pour fabriquer tout ou partie d'un médicament inhalable destiné à traiter ou à prévenir une déficience ou une défaillance d'organe périphérique choisi parmi les reins et le foie, et éventuellement les poumons, que cette déficience soit isolée ou multiple.

La déficience ou défaillance d'un organe se caractérise par une anomalie organique et/ou fonctionnelle de l'organe considéré, c'est-à-dire une incapacité ou une impossibilité de cet organe à présenter une fonctionnalité normale.

Cette déficience ou défaillance peut être transitoire, c'est-à-dire durer de moins d'une heure à plusieurs jours ou semaines, ou définitive.

La cause d'une déficience ou défaillance d'organe peut être en rapport notamment avec une anomalie/déficit de vascularisation, par exemple l'ischémie-reperfusion, ou d'oxygénation, à une infection localisée ou généralisée, une inflammation aiguë ou chronique localisée ou généralisée, des phénomènes d'auto-immunité primaires ou secondaires, à un traumatisme, à une organo-dégénérescence...

La déficience ou défaillance peut toucher concomitamment plusieurs organes et on parle alors de déficience multi-organes.

Il est connu de pouvoir traiter certaines déficiences ou défaillance affectant le cerveau à l'aide de gaz inhalés. Ainsi, le document EP-A-1158992 propose d'utiliser du xénon inhalé pour lutter contre les neuro-intoxications cérébrales, notamment ischémiques, qui se caractérisent par un dysfonctionnement cérébral d'un ou de plusieurs systèmes de neurotransmission.

Par ailleurs, le document EP-A-1541156 propose quant à lui d'utiliser de l'argon inhalé pour lutter contre lesdites neuro-intoxications cérébrales. Le potentiel neuroprotecteur de l'argon gazeux, administré seul ou en mélange avec du protoxyde d'azote, sur le développement et l'expression de la sensibilisation à la D-amphétamine a également été évalué.

Toutefois, les mécanismes d'action de l'argon sont encore mal connus et pourraient reposer sur une action agoniste des récepteurs GABA_{A}, notamment du site aux benzodiazépines, comme décrit par J. Abraini et al., Anesth Analg, 2003, 96:746-749.

Récemment, le capacité de neuroprotection de l'argon a été confirmée par Loetscher et al., Critical Care, 2009, 13 :R206 ;doi:10.1186/cc8214.

L'argon a été montré comme pouvant protéger des cellules cochléaires en culture isolée, c'est-à-dire les cellules dites chevelues issues de l'organe de Corti du rat, comme décrit par Yarin et al., Hear Res 2005, 201:1-9. Ces cellules sont assimilables aux neurones cérébraux, pour lesquels d'autres gaz, notamment le xénon, ont été montrés comme pouvant présenter un effet neuroprotecteur, comme rappelé par Ma et al., Br J Anaesth,2002, vol 98: 739-46.

En outre, le document EP-A-1651243 propose quant à lui d'utiliser des mélanges Xe/N₂O inhalé à cette fin, alors que le document DE-A-19991033704 propose des formulations liquides contenant du xénon dissout pour traiter les hypoxies et les ischémies cérébrales.

Comme on le voit, la plupart des traitements proposés visent à traiter des déficiences ou défaillance essentiellement cérébrales, c'est-à-dire qui affectent uniquement le cerveau.

Cependant, protéger uniquement le cerveau n'est pas suffisant. En effet, il convient également de pouvoir protéger les organes périphériques de toute défaillance, en particulier les organes vitaux comme le coeur, le foie, les reins...

Des études récentes ont montré que le xénon pouvait avoir une capacité à protéger des organes autres que le cerveau de conséquences de syndromes d'ischémie-reperfusion, par exemple à protéger le rein, comme décrit par Ma et al., J Am Soc Nephrol 2009, 20-4 :713-20, ou le coeur, comme enseigné par Schweibert et al., Eur J Anaesth 2010, Epub.

Par ailleurs, l'hélium, le néon et l'argon sont décrits par P. Pagel, dans Cardioprotection by Noble Gases, J. of Cardioth. & Vasc. Anaesth., vol. 24, n°1, 2010, comme ayant des propriétés protectrices cardiaques, lorsqu'il est administré à une teneur de 70% en volume, qui sont indépendantes de l'effet anesthésique.

En outre, le document DE-A-10319837 enseigne l'utilisation à des fins thérapeutiques ou prophylaxiques d'argon, de néon, d'hélium, de krypton, de xénon ou leurs mélanges des affections de l'oreille interne, notamment des otites, des lésions causées par le bruit...

Actuellement, il n'existe cependant pas de médicament réellement efficace permettant de protéger un ou plusieurs organes périphériques, autres que le cerveau ou le coeur, contre une déficience se caractérisant par une anomalie organique ou fonctionnelle de l'organe considéré, c'est-à-dire une incapacité ou une impossibilité de cet organe à présenter une fonctionnalité normale, en particulier d'organe périphérique tel les reins, le foie et les poumons, et ce quelle qu'en soit la cause.

Le problème est donc de proposer un traitement préventif et/ou curatif d'une déficience ou défaillance d'un (ou plusieurs) organe périphérique choisi parmi les reins, le foie et les poumons, et ce quelle qu'en soit la cause.

La solution de l'invention est alors une composition gazeuse contenant une quantité efficace d'argon gazeux pour une utilisation par inhalation pour prévenir ou traiter une déficience ou défaillance d'au moins un organe périphérique chez un patient, ledit organe périphérique étant choisi parmi le foie, et les reins.

Selon le cas, la composition gazeuse ou médicament gazeux inhalable de l'invention peut comprendre l'une ou plusieurs des caractéristiques suivantes :
- elle contient entre 15 et 80% en volume d'argon.
- elle contient au moins 30% en volume d'argon.
- elle contient moins de 75% en volume d'argon.
- la déficience d'organe résulte d'une anomalie organique et/ou fonctionnelle.
- la déficience d'organe concerne plusieurs organes.
- la déficience d'organe est transitoire, de préférence de moins d'une heure à plusieurs jours ou semaines, ou définitive.
- la déficience d'organe concerne plusieurs organes comprenant un ou plusieurs
   organes choisis parmi le foie et les reins et au moins un organe choisi parmi les poumons et par ailleurs le coeur.
- elle contient en outre de l'oxygène, de préférence au moins 21 % en volume d'oxygène.
- elle contient en outre un composé additionnel choisi dans le groupe formé par N₂O, Xe, He, Ne, NO, CO, H₂S et N₂.
- l'argon est administré au patient, une ou plusieurs fois par jour pendant une durée d'inhalation de quelques minutes à une ou plusieurs heures.
- elle est formée d'un mélange gazeux contenant, en outre, de l'oxygène, de l'azote ou leurs mélanges, en particulier de l'air.
- elle est formée d'un mélange gazeux binaire constitué d'argon et d'oxygène pour le reste ou un mélange ternaire constitué d'argon, d'azote et d'oxygène, de préférence le médicament gazeux est prêt à l'emploi.
- elle est conditionnée dans une bouteille de gaz.

L'invention porte aussi sur une utilisation d'argon gazeux pour fabriquer une composition médicamenteuse inhalable selon l'invention destinée à prévenir ou à traiter une déficience ou défaillance d'au moins un organe périphérique chez un patient, ledit au moins un organe périphérique étant choisi parmi le foie et les reins.

D'une façon générale, lors du traitement, l'administration d'argon au patient se fait par inhalation par exemple au moyen d'un ventilateur, d'un nébuliseur ou spontanément avec des bouteilles de gaz préconditionnées, raccordés à un masque facial ou nasal, ou des lunettes nasales.

La durée d'administration est fonction de la durée de la déficience / défaillance ou du risque de défaillance, choisie au cas par cas affectant le patient considéré, par exemple l'argon peut être administré pendant une durée d'administration de quelques minutes à quelques dizaines de minutes, voire d'heures, par exemple moins d'une heure, à une fréquence pouvant atteindre une à plusieurs fois par jour et sur une durée totale de traitement de un ou plusieurs jours, semaines, mois ou années.

L'argon ou mélange gazeux à base d'argon est préférentiellement conditionné en bouteille de gaz sous pression ou sous forme liquide, par exemple dans une bouteille d'un à plusieurs litres (contenant en eau) et à une pression comprise entre 2 et 300 bar.

L'argon ou mélange gazeux à base d'argon peut se présenter sous forme « prêt à l'emploi », par exemple en pré-mélange avec de l'oxygène, ou alors être mélangé sur site lors de son utilisation, notamment avec de l'oxygène et éventuellement un autre composé gazeux, par exemple de l'azote.

Dans le cadre de l'invention, le patient est un être humain, c'est-à-dire un homme ou une femme, y compris les enfants, les adolescents ou tout autre groupe d'individus, par exemple les nouveaux-nés.

### Exemple

Les essais suivants ont été réalisés pour démontrer que l'argon présente un effet protecteur sur la conservation d'un organe périphérique, à savoir ici un rein.

Pour ce faire, on a utilisé des reins isolés de rats destinés à une transplantation.

Dans cette étude, les effets de l'argon gazeux ont été étudiés sur les caractéristiques fonctionnelles et morphologiques des reins isolés.

120 rats Wistar, pesant entre 200 g et 250 g ont été utilisés en respectant le Guide pour l'utilisation et soins d'animaux de laboratoire, publié par The National Institutes of Health.

Les animaux ont opérés sous anesthésie générale et les reins gauches ont été prélevés puis conservés dans plusieurs atmosphères gazeuses, à savoir :
- Groupe A (selon invention) : argon (100% en volume)
- Groupe B (comparatif) : xénon (100% vol.)
- Groupe C (1^{er} contrôle) : air (i.e. mélange essentiellement N₂/O₂)
- Groupe D (2^{e} contrôle) : azote

Les reins d'un rat y sont conservés pendant 6 heures avant d'être greffés sur un autre rat anesthésie selon les conditions décrites antérieurement. Les rats sont gardés en observation pendant 14 jours.

Afin de réaliser une évaluation de l'efficacité de la protection engendrée par les différents gaz testés, on a examiné la fonction rénale et la morphologie rénale des reins greffés.

La fonction rénale a été évaluée par le calcul de la clearance de la créatinine estimée et rapportée à 100g de poids et par la mesure de l'albuminurie des 24 heures.

La morphologie rénale a été évaluée par histologie standard et histochimie à l'aide d'anticorps anti-active caspase-3 et anti-CD10.

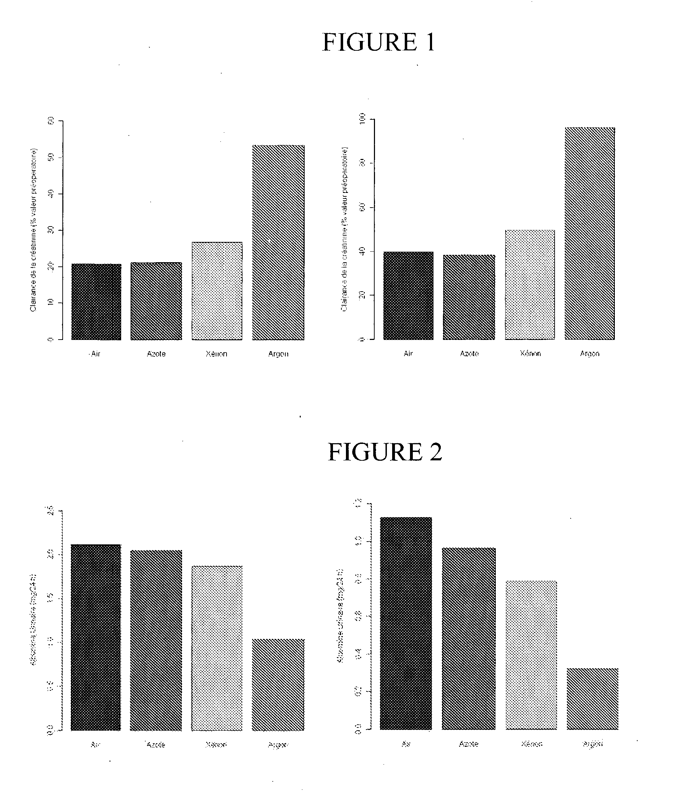

Les résultats obtenus pour ce qui concerne la fonction rénale sont schématisés sur les Figures 1 et 2 qui représentent :
- Figure 1 : Clearance de la Créatinine à jours J7 et J14
- Figure 2 : Albuminurie à jours J7 et J14

Comme on le constate, la Figure 1 montre clairement que l'argon est particulièrement efficace en ce qu'il a préservé une bonne clearance de la créatinine des reins greffés du Groupe A. La mesure de la clearance de la créatinine est le paramètre biologique principal permettant une évaluation de la fonction rénale.

A l'inverse, pour les Groupes B, C et D, la clairance de la créatinine est très faible pour les groupes traités par l'azote et l'air, et un peu meilleur avec le xénon, mais significativement plus faible comparé à l'argon. Ainsi, la fonction d'excrétion du rein est-elle préservée dans des conditions optimales avec l'argon.

Par ailleurs, la Figure 2 montre que dans le groupe Argon, l'albuminurie (présence d'albumine dans les urines qui normalement n'en contiennent pas et qui signale une destruction importante de la structure glomérulaire du rein) est moitié moindre dans le Groupe A que dans les autres groupes notamment comparés à l'azote et à l'air.

Ceci démontre que l'argon protège la structure du rein, ce qui sera confirmé par la suite de l'expérience (histologie).

Concernant la morphologie rénale évaluée par histologie standard, les reins des Groupes C et D contrôles (air et azote) montrent une lyse cellulaire avec une nécrose de coagulation sub-confluente.

Près d' 1/3 des reins du Groupe B (xénon) présentent des lésions du même type, mais aucun rein du Groupe A sous atmosphère argon ne présentait de signe de lyse cellulaire ou de nécrose.

De plus, concernant la morphologie rénale évaluée par histochimie, les organes du Groupe A (Ar selon l'invention) apparaissent mieux conservés que ceux des autres groupes. Le marquage CD10 est très bien préservé, ce qui équivaut à une conservation de la structure fonctionnelle tubulaire, alors que le marquage caspase-3 est discret et focalisé, ce qui montre une dégénérescence cellulaire très réduite, à l'opposé de ce qui est retrouvé dans les groupes xénon (Groupe B) et surtout contrôles (Groupe C et D).

Au final, les reins conservés sous argon conformément à l'invention conservent leurs capacités fonctionnelles, ainsi que leur intégrité physiques (au sens cellulaire et tissulaire) puisque l'argon diminue les lésions d'ischémie-reperfusion et améliore la fonction rénale.

Le mécanisme d'action par lequel l'argon est capable de protéger ces organes, notamment rein, coeur, foie, poumons lors d'une déficience aiguë ou chronique, isolée ou multiple, caractérisée par une anomalie organique et/ou fonctionnelle, transitoire ou définitive n'est pas encore établi avec certitude. Toutefois, on peut penser que l'argon pourrait agir via le maintien d'une vascularisation, oxygénation, homéostasie efficace et/ou une régulation des mécanismes d'apoptose cellulaire, c'est-à-dire les mécanismes conduisant à la mort cellulaire...

Dans tous les cas, ces essais montrent que l'argon peut être utilisé en tant que médicament inhalable pour prévenir ou traiter une déficience ou défaillance d'un ou plusieurs organes périphériques chez un patient, en particulier le foie, les reins, les poumons, que la défaillance d'organe soit due à une infection localisée ou généralisée, une inflammation aiguë ou chronique localisée ou généralisée, des phénomènes d'auto-immunité primaires ou secondaires, à un traumatisme, à une organo-dégénérescence ou une autre cause.

## Revendications

1. Composition gazeuse contenant une quantité efficace d'argon gazeux pour une utilisation par inhalation pour prévenir ou traiter une déficience ou défaillance d'au moins un organe périphérique chez un patient, ledit au moins un organe périphérique étant choisi parmi le foie et les reins.

2. Composition selon la revendication précédente, **caractérisée en ce qu'**elle contient entre 15 et 80% en volume d'argon.

3. Composition selon l'une des revendications précédentes, **caractérisée en ce qu'**elle contient au moins 30% en volume d'argon.

4. Composition selon l'une des revendications précédentes, **caractérisée en ce qu'**elle contient moins de 75% en volume d'argon.

5. Composition selon l'une des revendications précédentes, **caractérisée en ce que** la déficience ou défaillance d'organe résulte d'une anomalie organique et/ou fonctionnelle.

6. Composition selon l'une des revendications précédentes, **caractérisée en ce que** la déficience ou défaillance d'organe concerne plusieurs organes.

7. Composition selon l'une des revendications précédentes, **caractérisée en ce que** la déficience ou défaillance d'organe est transitoire, de préférence de moins d'une heure à plusieurs jours ou semaines, ou définitive

8. Composition selon l'une des revendications précédentes, **caractérisée en ce que** la déficience ou défaillance d'organe concerne plusieurs organes, au moins l'un des organes étant choisi parmi le foie et les reins, et au moins un autre organe étant choisi parmi le coeur et les poumons.

9. Composition selon l'une des revendications précédentes, **caractérisée en ce qu'**elle contient en outre de l'oxygène, de préférence au moins 21% en volume d'oxygène.

10. Composition selon l'une des revendications précédentes, **caractérisée en ce qu'**elle contient en outre un composé additionnel choisi dans le groupe formé par N₂O, Xe, He, Ne, NO, CO, H₂S et N₂.

## Patentansprüche

1. Gasförmige Zusammensetzung, die eine wirksame Menge gasförmigen Argons enthält, für eine Verwendung mittels Inhalation, um eine Dysfunktion oder Insuffizienz mindestens eines peripheren Organs bei einem Patienten zu verhindern oder zu behandeln, wobei das mindestens eine periphere Organ aus der Leber und den Nieren ausgewählt ist.

2. Zusammensetzung nach dem vorhergehenden Anspruch, **dadurch gekennzeichnet, dass** sie zwischen 15 und 80 Volumen-% Argon enthält.

3. Zusammensetzung nach einem der vorhergehenden Ansprüche, **dadurch gekennzeichnet, dass** sie mindestens 30 Volumen-% Argon enthält.

4. Zusammensetzung nach einem der vorhergehenden Ansprüche, **dadurch gekennzeichnet, dass** sie weniger als 75 Volumen-% Argon enthält.

5. Zusammensetzung nach einem der vorhergehenden Ansprüche, **dadurch gekennzeichnet, dass** die Organdysfunktion oder -insuffizienz aus einer Organ- und/oder Funktionsanomalie resultiert.

6. Zusammensetzung nach einem der vorhergehenden Ansprüche, **dadurch gekennzeichnet, dass** die Organdysfunktion oder -insuffizienz mehrere Organe betrifft.

7. Zusammensetzung nach einem der vorhergehenden Ansprüche, **dadurch gekennzeichnet, dass** die Organdysfunktion oder -insuffizienz vorübergehend ist, vorzugsweise weniger als eine Stunde bis zu mehreren Tagen oder Wochen oder dauerhaft anhält.

8. Zusammensetzung nach einem der vorhergehenden Ansprüche, **dadurch gekennzeichnet, dass** die Organdysfunktion oder -insuffizienz mehrere Organe betrifft, wobei mindestens eines der Organe aus der Leber und den Nieren ausgewählt ist und mindestens ein anderes Organ aus dem Herzen und den Lungen ausgewählt ist.

9. Zusammensetzung nach einem der vorhergehenden Ansprüche, **dadurch gekennzeichnet, dass** sie außerdem Sauerstoff, vorzugsweise mindestens 21 Volumen-% Sauerstoff, enthält.

10. Zusammensetzung nach einem der vorhergehenden Ansprüche, **dadurch gekennzeichnet, dass** sie außerdem eine zusätzliche Verbindung enthält, die aus der Gruppe bestehend aus N₂O, Xe, He, Ne, NO, CO, H₂S und N₂ ausgewählt ist.

## Claims

1. Gaseous composition containing an effective amount of gaseous argon for use by inhalation to prevent or treat a deficiency or failure of at least one peripheral organ in a patient, said at least one peripheral organ being selected from between the liver and the kidneys.

2. Composition according to the preceding claim, **characterised in that** it contains between 15 and 80 % by volume of argon.

3. Composition according to any of the preceding claims, **characterised in that** it contains at least 30 % by volume of argon.

4. Composition according to any of the preceding claims, **characterised in that** it contains less than 75 % by volume of argon.

5. Composition according to any of the preceding claims, **characterised in that** the organ deficiency or failure results from an organic and/or a functional abnormality.

6. Composition according to any of the preceding claims, **characterised in that** the organ deficiency or failure affects a plurality of organs.

7. Composition according any of the preceding claims, **characterised in that** the organ deficiency or failure is either transient, preferably lasting from less than one hour to a plurality of days or weeks, or definitive.

8. Composition according to any of the preceding claims, **characterised in that** the organ deficiency or failure affects a plurality of organs, at least one of the organs being selected from between the liver and the kidneys, and at least one other organ being selected from between the heart and the lungs.

9. Composition according to any of the preceding claims, **characterised in that** it further contains oxygen, preferably at least 21 % by volume of oxygen.

10. Composition according to any of the preceding claims, **characterised in that** it further contains an additional compound selected from within the group made up of N₂O, Xe, He, Ne, NO, CO, H₂S and N₂.
